# EUROPEAN PATENT SPECIFICATION

(11) **EP 2 804 597 B1**
(45) Date of publication and mention of the grant of the patent: **20.04.2016**
(21) Application number: 13707041.3
(22) Date of filing: 15.01.2013
(51) Int. Cl.: A61K 31/167, A61K 47/12, A61K 47/18, A61K 47/26, A61K 9/00, A61P 29/02, A61K 9/08

(54) **AQUEOUS PARACETAMOL COMPOSITION FOR INJECTION**
WÄSSRIGE PARACETAMOLZUSAMMENSETZUNG ZUR INJEKTION
COMPOSITION AQUEUSE DE PARACÉTAMOL POUR INJECTION

(30) Priority: 16.01.2012 ES 201230058
(43) Date of publication of application: 26.11.2014
(73) Proprietor: Novocat Farma, S.A., 08191 Rubí (ES)
(72) Inventor: RIZZO TAMARO, Adriana, E-08191 Rubí (ES)
(74) Representative: Gallego Jiménez, José Fernando
(86) International application number: PCT/IB2013/050370
(87) International publication number: WO 2013/108180

(56) References cited:
- EP-A1- 2 377 516
- WO-A1-2009/064928
- WO-A1-2011/071400
- WO-A2-2009/081283
- US-A1- 2004 054 012

## Description

### Technical field.

The present invention relates to a stable aqueous paracetamol formulation, suitable for its administration by injection.

### Background art.

Paracetamol or acetaminophen is a drug of recognized analgesic and antipyretic properties, and constitutes the drug of first choice for the treatment of pain and fever conditions in many groups of patients. In particular, paracetamol is a drug widely used in hospitals, where the compositions for intravenous administration are especially useful, because this route allows a more rapid onset of the therapeutic action, and also a greater efficacy and a longer analgesic duration in acute pain management in the hospital setting. Therefore, in this context, it is crucial to have stable liquid formulations of paracetamol which are suitable for intravenous administration.

However, the preparation of these kinds of solutions, ready for use by intravenous infusion, shows a number of difficulties, mainly due to the fact that paracetamol and, in general, phenolic active ingredients, are unstable in aqueous solution, since they are sensitive to oxidation by the oxygen dissolved in the medium. On the one hand, this causes problems of loss of activity of the solution, due to the decrease of the effective amount of active ingredient, and on the other hand, it also causes problems derived from the possible toxicity of the degradation products formed.

Specifically, it is known that paracetamol is easily hydrolyzed to 4-aminophenol and the latter is also easily oxidized, to form benzoquinoneimine derivatives which are hepatotoxic, as disclosed, for example, in the article Fairbrother J.E., Acetaminophen, Analytical Profiles of Drug Substances, 1974, 3, 1-109.

The presence of these degradation products can often be seen with the naked eye, since they give colour to the solution which, in optimal conditions, must be totally colourless.

It has been proven that the rate of hydrolysis of paracetamol in aqueous solutions is pH dependent, so the formation of 4-aminophenol can be minimized by adjusting pH to a value approximately comprised between 4.5 and 6, as disclosed in the article Koshy et al., Stability of aqueous solutions of N-acetyl-p-aminophenol, J. Phar. Sci., 1961, 50 (2), 113-118.

Thus, there exist several common practices aimed at achieving stable aqueous solutions of paracetamol. On the one hand, water with low oxygen content is typically used, which can be achieved by procedures that are well known in the field of pharmaceutical technology, for example, by heating or by bubbling an inert gas. On the other hand, it is also common to use buffering or buffer systems, to obtain a stable pH of the solution within the above mentioned approximate range of 4.5-6, where the paracetamol degradation is lower.

However, these procedures, by themselves, are insufficient to achieve aqueous paracetamol solutions that are stable for a prolonged time. Therefore, this has been the objective of many studies in the field of pharmaceutical research, as evidenced by the large number of different proposals that can be found in the prior art.

Thus, for example, in the international patent application WO-A-98/05314 there are disclosed aqueous paracetamol formulations stabilized by the addition of a free radical capturing agent, removing the oxygen dissolved by bubbling nitrogen, and adjusting pH to a range between 4 and 8.

In the international patent application WO-A-01/93830 it is disclosed the preparation of stable liquid paracetamol compositions which contain cysteine as antioxidant agent and citrate buffer and in which low pressure packaging conditions are kept.

In the international patent application WO-A-02/072080 there are disclosed injectable paracetamol solutions which are stable to storage and which comprise the active ingredient and an antioxidant agent selected from ascorbic acid, and compounds comprising thiol groups. Also, it is disclosed that the solutions have a pH value comprised between 5.5 and 6.5 and have low dissolved oxygen content.

In the international patent application WO-A-2004/071502, it is disclosed an injectable liquid formulation of paracetamol comprising water, monosodium phosphate and disodium phosphate to obtain a buffered pH comprised between 4.5 and 6.5, sodium chloride as isotonic agent, and the dimer of paracetamol as antioxidant agent, which is formed during the sterilizing process of the vials at high temperature, for example at 121º C for 15 minutes.

In the European patent application EP-A-1752139, it is disclosed an aqueous paracetamol composition which comprises an antioxidant agent selected form the group consisting of ascorbic acid, N-acetyl-L-cysteine, and other stabilizing compounds containing the -SH group, which is essentially free of organic solvents and buffering systems, and which contains less than 1 mg/l of oxygen.

In the international patent application WO-A-2008/009756, aqueous solutions of paracetamol are disclosed, which comprise as stabilizing agent a substance able to react with phenolates, such as for example, reducing sugars such as glucose, galactose, or fructose, or chemical species containing sulphur in an oxidation state less than +6, as for example, sodium formaldehyde sulfoxylate, sulfites or thiourea.

In the international patent application WO-A-2008/135601 it is disclosed the preparation of an aqueous paracetamol formulation stable to oxidation which contains water with low oxygen content, disodium phosphate as a buffer to adjust the pH to a value comprised between 5 and 6 and mannitol as an isotonizing agent, and that is characterized because the temperature must remain between 60 ºC and 105 ºC throughout the whole preparation process.

In the international patent application WO-A-2009/081283, it is disclosed an aqueous paracetamol composition which contains mannitol, L-cysteine, povidone and monobasic sodium phosphate as excipients, and with a pH value adjusted to 5.5, with the distinctive feature that during the process sodium bicarbonate is dissolved until reaching a pH of 8, and then citric acid is added until a pH of 5.5, so that carbonic anhydride is generated which displaces the oxygen present in the solution.

In the international patent application WO-A-2009/064928 aqueous paracetamol compositions are disclosed which comprise a buffering system, an isotonizing agent, and that also have at last an antioxidant, such as for example, metabisulfite, acetylcysteine, methionine, reduced glutathione, ascorbic acid, or cysteine.

In the European patent application EP-A-2277546 aqueous paracetamol formulations are disclosed comprising hydroxypropylcyclodextrin as solubilizing-stabilizing agent, EDTA and monothioglycerol as stabilizing agents, sodium chloride and a buffer agent such as, for example, phosphate, citrate, or tartrate.

In the international patent application WO-A-2011/018522, there are disclosed aqueous liquid formulations of paracetamol comprising mannitol as isotonic agent; a buffer agent as, for example, phosphate, citrate, acetate, bicarbonate, or tartrate; and cysteine hydrochloride as antioxidant agent.

In general, the different proposals found in the prior art consist on relatively complex compositions, usually requiring the incorporation of more than one component having antioxidant, antiradical, or stabilizing characteristics; or else require complex preparation processes.

Thus, there remains a need to provide an alternative aqueous formulation of paracetamol which is stable and suitable for its intravenous administration, which is both easy to prepare and does not require a complex composition to achieve stability.

### Object of the invention.

The object of the present invention is an aqueous paracetamol composition for injection.

Another aspect of the invention is a process for the preparation of said composition.

Another aspect of the invention is the use of tromethamine for the stabilization of injectable aqueous paracetamol compositions.

Another aspect of the invention is the paracetamol composition for the treatment of pain and fever conditions.

### Detailed description of the invention.

The object of the present invention is an aqueous composition for injection comprising:
- paracetamol as active ingredient,
- an isotonizing agent,
- an effective amount of a buffering system to obtain a pH comprised between 4.5 and 7.5,
wherein the buffering system comprises a carboxylic acid and tromethamine.

The authors of the present invention have observed that, surprisingly, the addition of tromethamine to liquid paracetamol formulations for injection provides an enhanced stability thereof.

The composition of the invention is an aqueous paracetamol solution that is optimal for its use for injection for the treatment of pain and fever conditions. Therefore, another aspect of the invention is the composition of the invention for the treatment of pain and fever conditions, and the use of the composition of the invention for the preparation of a medicament for the treatment of pain and fever conditions.

### The active ingredient

Paracetamol, also known as acetaminophen, is the INN (International Nonproprietary Name) of the product N-(4-hydroxyphenyl)ethanamide.

Paracetamol can be obtained, for example, from 4-aminophenol, as disclosed in the U.S. patent US2998459.

The liquid paracetamol formulation object of the present invention can be in diluted form with a content of active ingredient comprised between 2 mg/ml and 50 mg/ml or in concentrated form with a content of active ingredient comprised between 60 mg/ml and 350 mg/ml, and more preferably between 100 mg/ml and 250 mg/ml.

Preferably the concentration of the active ingredient is comprised between 5 mg and 50 mg of paracetamol per ml of solution, more preferably comprised between 8 mg and 15 mg of paracetamol per ml of solution, and still more preferably between 9 mg and 12 mg of paracetamol per ml of solution.

Preferably, the liquid formulation of the present invention is dosed either in vials containing between 0.4 g and 0.6 g of paracetamol, preferably 0.5 g of paracetamol per vial; or in vials containing between 0.9 g and 1.1 g of paracetamol, preferably 1 g of paracetamol per vial.

### Buffering system

The composition of the invention comprises an effective amount of a buffering system that allows obtaining a solution with an essentially stable pH, with a value comprised between 4.5 and 7.5, preferably between 5.5 and 6.5, and more preferably between 5.7 and 6.1.

The buffering system of the composition of the invention comprises a carboxylic acid and tromethamine. That means that the buffering system is formed by a carboxylic acid and the salt of said acid with tromethamine.

Optionally, the buffering system may contain other buffering substances, besides the pair carboxylic acid-tromethamine, as for example, phosphate buffer.

In a particularly preferred embodiment the buffer system essentially consists of a carboxylic acid and tromethamine, i.e., it does not include any additional buffer system.

### Tromethamine

Tromethamine, trometamol, or TRIS are some of the conventional names that receives the product 2-amino-2-hydroxymethyl-propane-1,3-diol.

Tromethamine can be obtained commercially from various companies as, for example, Sigma-Aldrich.

Tromethamine can also be prepared as described in the article Johnson et al., The utilization of aliphatic nitro compounds. V. Reduction of nitro alcohols and nitro glycols to the corresponding amines, J. Org. Chem., 1943, 8 (1), 7-9.

Tromethamine is a substance of basic character that is added to the aqueous composition of the invention, together with a carboxylic acid, to form a buffered solution with a pH that remains essentially stable in the range comprised between 4.5 and 7.5, preferably between 5.5 and 6.5, and more preferably between 5.7 and 6.1.

Within the scope of the present invention tromethamine is used preferably in a concentration comprised between 0.1 mg and 3 mg of tromethamine per ml of solution, more preferably comprised between 0.2 mg and 1 mg of tromethamine per ml of solution, and still more preferably comprised between 0.3 mg and 0.7 mg of tromethamine per ml of solution.

### Carboxylic acid

The carboxylic acid that is part of the buffering system is a carboxylic acid pharmaceutically acceptable and compatible with intravenous administration.

Carboxylic acids are organic acids that have at least one carboxylic group (-COOH) in their structure. They can be, for example, monocarboxylic (only one carboxylic group), dicarboxylic (two carboxylic groups) or tricarboxylic (three carboxylic groups).

The carboxylic acids appropriate to be used in the compositions of the present invention can be selected from the group consisting of lactic acid, acetic, sorbic, gluconic, tartaric, malic, succinic, fumaric, citric acid, and mixtures thereof.

In a preferred embodiment, the carboxylic acid is selected from citric acid, lactic acid and tartaric acid, and mixtures thereof. In a still more preferred embodiment, the carboxylic acid is citric acid.

The carboxylic acid can also be added to the composition of the invention in any of its hydrated forms as, for example, citric acid monohydrate.

The buffering system that is part of the composition of the invention can also be prepared by mixing the carboxylic acid and its alkaline salt and tromethamine, so that the carboxylic acid salt with tromethamine is formed in the system.

The carboxylic acid, together with tromethamine, form a buffering system that allows to obtain a solution with a pH which remains essentially stable in the range comprised between 4.5 and 7.5, preferably between 5.5 and 6.5, and more preferably between 5.7 and 6.1.

The final pH of the solution can be finally adjusted by the addition of an acid or a base, usually a diluted aqueous hydrochloric acid solution, for example, of 10%, or a diluted aqueous sodium hydroxide solution, for example, of 4%.

Preferably, tromethamine and the carboxylic acid are used in a proportion characterized by the molar ratio tromethamine:carboxylic groups, so that this ratio is comprised between 1.2:1 and 3:1, more preferably comprised between 1.5:1 and 2.5:1, and still more preferably comprised between 1.9:1 and 2.3:1.

Thus, for example, if the carboxylic acid is lactic acid, for a composition comprising between 0.1 mg and 3 mg of tromethamine per ml of solution, preferably between 0.02 mg and 1.85 mg of lactic acid per ml of solution are used. If tartaric acid is used, preferably between 0.02 mg and 1.55 mg of tartaric acid per ml of solution are used.

In a preferred embodiment, the composition of the invention comprises citric acid. Preferably, the composition comprises between 0.02 mg and 1.32 mg of citric acid per ml of solution, more preferably between 0.08 mg and 0.8 mg of citric acid per ml of solution, and still more preferably between 0.1 mg and 0.2 mg of citric acid per ml of solution.

### Isotonizing agent

The paracetamol composition for injection of the invention or injectable paracetamol composition is intended to its administration by intravenous infusion.

As it is well known to those skilled in pharmaceutical technology, the solutions for injection must accomplish a number of requirements. Among them, that such solutions are isotonic to blood, and that is why the compositions of the invention incorporate an isotonizing agent.

The isotonizing agents suitable to be used in the present invention can be selected form the group formed by sodium chloride, potassium chloride, ethanol, polyethylene glycol, propylene glycol, sorbitol, inositol, mannitol, glycerol, glucose, fructose, dextrose, mannose, sucrose, lactose, maltose, and mixtures thereof. Preferably the isotonizing agent is selected from sodium chloride and mannitol.

In a preferred embodiment, the composition of the invention comprises between 1 mg and 15 mg of sodium chloride per ml of solution, preferably between 5 mg and 10 mg, and more preferably between 5.5 mg and 8 mg.

In another preferred embodiment, the composition of the invention comprises between 10 mg and 50 mg of mannitol per ml of solution, preferably between 15 mg and 45 mg, and more preferably between 20 mg and 40 mg.

### Water for injection

The composition of the invention is prepared with water for injection, which can be obtained, for example, by sterilization of distilled water.

The water for injection for the compositions of the present invention preferably has low oxygen content.

The water for injection suitable to be used in the composition of the invention has preferably an oxygen content equal to or less than 0.5 ppm at 25º C of temperature and 1 atm of pressure, and more preferably equal to or less than 0.3 ppm at 25º C of temperature and 1 atm of pressure.

The water for injection with low oxygen content can be obtained, for example, by heating distilled water in a reactor at a temperature of 100º C under 1 atm of pressure, allowing the vapour to escape through the valve, for enough time, for example, 20 minutes until obtaining water with the desired oxygen content. It can also be obtained by bubbling an inert gas as, for example, nitrogen, carbonic anhydride or argon.

### Preparation of the compositions of the invention

Another aspect of the invention is a process for the preparation of an aqueous paracetamol composition for injection.

The process for the preparation of the compositions of the invention comprises the following steps:
1) dissolving paracetamol, tromethamine, carboxylic acid and isotonizing agent in water for injection; and
2) adjusting the pH, if necessary, by adding acid or base to a value comprised between 4.5 and 7.5.

Usually the water for injection is firstly prepared, preferably with an oxygen content equal to or less than 0.5 ppm, more preferably equal to or less than 0.3 ppm, by heating distilled water at 100º C and at 1 atm of pressure for 20 minutes, until obtaining water at 25º C and at 1 atm of pressure with the required oxygen content.

Next, the water for injection is placed in a reactor provided with stirring means and the different components of the formulation are consecutively incorporated: paracetamol, tromethamine, carboxylic acid and the isotonizing agent, maintaining the mixture continuously under stirring in order to achieve the complete dissolution of the components.

If necessary, the pH of the obtained solution is adjusted to a value comprised between 4.5 and 7.5 by using a diluted aqueous solution of hydrochloric acid, for example, of 10%, or a diluted aqueous solution of sodium hydroxide, for example, of 4%.

Finally, it is adjusted with water of injection until obtaining the desired concentration of the active ingredient.

The paracetamol solution obtained is sterilized, for example, by filtration. Subsequently, the obtained solution can be dosed in containers, for example, sterile vials preferably of 50 ml or 100 ml, which are closed with a stopper, preferably previously filling with nitrogen the empty space between the solution and the stopper.

A particularly preferred embodiment of the invention is a paracetamol composition comprising:
- between 5 mg/ml and 50 mg/ml, preferably between 8 mg/ml and 15 mg/ml, and more preferably between 9 mg/ml and 12 mg/ml of paracetamol,
- between 0.1 mg/ml and 3 mg/ml, preferably between 0.2 mg/ml and 1 mg/ml, and more preferably between 0.3 mg/ml and 0.7 mg/ml of tromethamine,
- between 0.02 mg/ml and 1.32 mg/ml, preferably between 0.08 mg/ml and 0.8 mg/ml, and more preferably between 0.1 mg/ml and 0.2 mg/ml of citric acid, and
- between 1 mg/ml and 15 mg/ml, preferably between 5 mg/ml and 10 mg/ml, and more preferably between 5.5 mg/ml and 8 mg/ml of sodium chloride.

Such composition is adjusted to a pH comprised between 4.5 and 7.5, preferably between 5.5 and 6.5, and more preferably between 5.7 and 6.1, occasionally by using a diluted aqueous hydrochloric acid solution or a diluted aqueous sodium hydroxide solution. The units mg/ml relate to the weight of each component per ml of solution.

Another particularly preferred embodiment of the invention is a paracetamol composition comprising:
- between 5 mg/ml and 50 mg/ml, preferably between 8 mg/ml and 15 mg/ml, and more preferably between 9 mg/ml and 12 mg/ml of paracetamol,
- between 0.1 mg/ml and 3 mg/ml, preferably between 0.2 mg/ml and 1 mg/ml, and more preferably between 0.3 mg/ml and 0.7 mg/ml of tromethamine,
- between 0.02 mg/ml and 1.32 mg/ml, preferably between 0.08 mg/ml and 0.8 mg/ml, and more preferably between 0.1 mg/ml and 0.2 mg/ml of citric acid, and
- between 10 mg/ml and 50 mg/ml, preferably between 15 mg/ml and 45 mg/ml, and more preferably between 20 mg/ml and 40 mg/ml of mannitol.

Such composition is adjusted to a pH comprised between 4.5 and 7.5, preferably between 5.5 and 6.5, and more preferably between 5.7 and 6.1, occasionally by using a diluted aqueous hydrochloric acid solution or a diluted aqueous sodium hydroxide solution. The units mg/ml relate to the weight of each component per ml of solution.

### Stability tests

The paracetamol composition of the invention shows a notable stability even in accelerated stability conditions.

In Example 3 the results of a stability study are shown where two compositions according to the present invention are compared to two reference formulations containing cysteine as antioxidant agent, and it is observed that the former show higher stability than the formulations with cysteine.

In Example 4 the results of a comparative stability test are shown between a formulation according to the invention, which contains tromethamine and an analogous formulation without tromethamine. It is also confirmed in this example, that the composition with tromethamine is remarkably more stable that the composition without tromethamine.

That is why another aspect of the invention is the use of tromethamine for the stabilization of injectable aqueous solutions of paracetamol.

The following examples are intended to illustrate the invention but they should not be considered as limiting it.

### Examples

### Example 1: Aqueous paracetamol composition with sodium chloride as isotonizing agent

An aqueous paracetamol solution was prepared. using the components detailed in Table I:

**TABLE I**

| **Ingredient** | **Amount (g/100 ml)** |
|---|---|
| Paracetamol | 1 |
| Tromethamine | 0.0515 |
| NaCl | 0.70 |
| Citric acid monohydrate | 0.014 |
| HCl 10% | q.s. to pH=6.0 |
| NaOH 4% | q.s. to pH=6.0 |
| water for injection | q.s. |

Water for injection with an oxygen content of less than 0.3 ppm was prepared by heating distilled water to 100º C and at 1 atm of pressure for 20 minutes.

A part of the water for injection obtained was placed in a secondary reactor, approximately a 8%, and it was boiled for 5 minutes. The rector was closed and the water was left to cool to 40º C.

Next, the stirrer was connected and paracetamol, tromethamine, citric acid monohydrate and sodium chloride were consecutively added, waiting 5 minutes between each addition. The solution obtained was transferred to the main reactor, with the rest of the water for injection.

The pH was determined and it was adjusted to 6.0 by using diluted aqueous hydrochloric acid and sodium hydroxide solutions. It was completed with additional water for injection until obtaining the required concentration of paracetamol (10 mg/ml), and it was stirred for 20 more minutes. The solution was sterilized by filtration and filled into sterile vials of 100 ml, filling the empty space with nitrogen before inserting the rubber stopper.

### Example 2: Aqueous paracetamol composition with mannitol as isotonizing agent

An aqueous paracetamol solution was prepared, using the components detailed in the Table II:

**TABLE II**

| **Ingredient** | **Amount (g/100 ml)** |
|---|---|
| Paracetamol | 1 |
| Tromethamine | 0.0515 |
| Mannitol | 3.36 |
| Citric acid monohydrate | 0.014 |
| HCl 10% | q.s. to pH=6.0 |
| NaOH 4% | q.s. to pH=6.0 |
| water for injection | q.s. |

To prepare the paracetamol solution of Example 2 it was followed an analogous procedure to the one described in Example 1, but in this case the isotonizing agent selected was mannitol, instead of sodium chloride.

### Reference Example 1: Aqueous paracetamol composition with cysteine as stabilizing agent

For comparative purposes, an aqueous paracetamol composition analogous to the commercial product Perfalgan® was prepared, containing cysteine as antioxidant agent. To this end, the components set out in the Table III were used:

**TABLE III**

| **Ingredient** | **Amount (g/100 ml)** |
|---|---|
| Paracetamol | 1 |
| Cysteine | 0.1 |
| Mannitol | 3.85 |
| Disodium phosphate dihydrate | 0.13 |
| HCl 10% | q.s. to pH=6.0 |
| NaOH 1M | q.s. to pH=6.0 |
| water for injection | q.s. |

For the preparation of this composition, water for injection with oxygen content of less than 0.3 ppm was prepared as described in Example 1 and placed in a reactor.

Next, the stirrer was connected and then paracetamol, cysteine, disodium phosphate dihydrate and mannitol were successively added, waiting 5 minutes between each addition.

The pH was determined and it was adjusted to 6.0 by using diluted aqueous hydrochloric acid and sodium hydroxide solutions. Additional water for injection was added to obtain the required paracetamol concentration (10 mg/ml), and it was stirred for 20 minutes.

Finally, the solution was sterilized by filtration and filled into sterile vials of 100 ml, filling the empty space with nitrogen before inserting the rubber stopper.

### Reference Example 2: Aqueous paracetamol composition with mannitol as isotonizing agent

To assess the effect of tromethamine in the stability of the compositions of the invention, an aqueous paracetamol composition was prepared which was analogous to the one of Example 2, but without tromethamine. The components detailed in Table IV were used:

**TABLE IV**

| **Ingredient** | **Amount (g/100 ml)** |
|---|---|
| Paracetamol | 1 |
| Mannitol | 3.36 |
| Sodium citrate | 0.0355 |
| Citric acid monohydrate | 0.014 |
| HCl 10% | q.s. to pH=6.0 |
| NaOH 4% | q.s. to pH=6.0 |
| water for injection | q.s. |

For the preparation of this composition, water for injection with oxygen content of less than 0.3 ppm was prepared as described in Example 1 and placed in a reactor.

Next, the stirrer was connected and then paracetamol, sodium citrate, citric acid and mannitol were successively added, waiting 5 minutes between each addition. The pH of the solution was adjusted to a value of 6.0 by using a buffering system of citric acid/sodium citrate, and it was further adjusted with aqueous hydrochloric acid and sodium hydroxide solutions. Additional water for injection was added to obtain the required paracetamol concentration (10 mg/ml), and it was stirred for 20 minutes. The solution was sterilized by filtration and filled into sterile vials of 100 ml, filling the empty space with nitrogen before inserting the rubber stopper.

### Example 3: Comparative stability testing of the formulations of the invention with respect to formulations with cysteine

To check the stability of the compositions of the invention, a study was carried out where the formation of paracetamol degradation products was tested over a period of 6 weeks, for samples of the compositions of the invention prepared according to Examples 1 and 2, as well as, for comparative purposes, for two formulations with cysteine: the formulation of Reference Example 1 and the commercial product Perfalgan®, which contains paracetamol, mannitol, cysteine hydrochloride monohydrate, disodium phosphate dihydrate, sodium hydroxide, hydrochloric acid and water for injection.

The samples were analyzed at the beginning of the test, and after 1, 3 and 6 weeks. Each product was subjected to three temperature conditions: 30º C, 45º C and 60º C. For each test, the content of paracetamol and of its degradation products was determined by means of high performance liquid chromatography (HPLC).

The conditions used for the HPLC analytical determination were essentially based on the method disclosed in the European Pharmacopoeia, 6^{th} edition, for the determination of paracetamol and its impurities, comparing the relative area with an external paracetamol standard of known concentration.

4 degradation products were determined:
- 4-aminophenol (P1),
- 4-nitrophenol (P2),
- unidentified product recovered at a retention time of 0.8 minutes (P3), and
- unidentified product recovered at a retention time of 1.3 minutes (P4).

It was also performed a visual analysis of the samples to determine the appearance of colouring, using the following rating scale:
- colourless (0),
- slightly yellow (1),
- yellow (2),
- yellow-brown (3), and
- brown (4).

The results obtained in this study are shown in the Tables V to VIII.

In Table V, the results obtained at time 0, i.e. at the beginning of the test, are shown. It can be observed that all solutions were colourless, but the product Perfalgan showed the presence of 4-aminophenol (P1) degradation product:

**TABLE V**

| *Time 0* | *Colour* | *Paracetamol (mg)* | *Degradation products (%)* | | | | |
|---|---|---|---|---|---|---|---|
| | | | P1* | P2** | P3*** | P4**** | Total |
| Example 1 | 0 | 9.88 | - | - | - | - | - |
| Example 2 | 0 | 9.94 | - | - | - | - | - |
| Ref. Ex.1 | 0 | 8.73 | - | - | - | - | - |
| Perfalgan | 0 | 9.8 | 0.02 | - | - | - | 0.02 |

| | | | | | | | |
|---|---|---|---|---|---|---|---|
| * 4-aminophenol not detected (-): <0.002% ** 4-nitrophenol not detected (-): <0.01% *** P3 not detected (-): <0.02% **** P4 not detected (-): <0.005% | | | | | | | |

Table VI shows the results obtained after 1 week, at the three temperature conditions tested. It can be observed that the compositions of the invention (Example 1 and Example 2) remain colourless and without detectable degradation products. Reference Example 1 shows some colouring at 45º C and at 60º C, without degradation products detected. Perfalgan® samples show a higher colouring at these temperatures and detectable levels of 4-aminophenol (P1):

**TABLE VI**

| *Week 1* | T | *Colour* | *Paracetamol (mg)* | *Degradation products* | | | | |
|---|---|---|---|---|---|---|---|---|
| | | | | P1* | P2* | P3*** | P4**** | Total |
| Example 1 | 30º C | 0 | 9.92 | - | - | - | - | - |
| | 45º C | 0 | 9.98 | - | - | - | - | - |
| | 60º C | 0 | 9.89 | - | - | - | - | - |
| Example 2 | 30º C | 0 | 9.74 | - | - | - | - | - |
| | 45º C | 0 | 9.47 | - | - | - | - | - |
| | 60º C | 0 | 9.81 | - | - | - | - | - |
| Ref. Ex.1 | 30º C | 0 | 8.16 | - | - | - | - | - |
| | 45º C | 1 | 8.11 | - | - | - | - | - |
| | 60º C | 1 | 8.93 | - | - | - | - | - |
| Perfalgan | 30º C | 0 | 7.74 | 0.02 | - | - | - | 0.02 |
| | 45º C | 2 | 7.53 | 0.01 | - | - | - | 0.01 |
| | 60º C | 3 | 7.81 | - | - | - | - | - |

| | | | | | | | | |
|---|---|---|---|---|---|---|---|---|
| * 4-aminophenol not detected (-): <0.002% ** 4-nitrophenol not detected (-): <0.01% *** P3 not detected (-): <0.02% **** P4 not detected (-): <0.005% | | | | | | | | |

Table VII shows the results obtained after 3 weeks, at the three temperature conditions. It can be observed that the compositions of the invention (Example 1 and Example 2) remain colourless and without detectable degradation products, like Reference Example 1, albeit the latter shows some colouring at 45º C and 60 ºC. Perfalgan® samples show significant colouring and detectable levels of degradation products:

**TABLE VII**

| *Week 3* | T | *Colour* | *Paracetamol (mg)* | *Degradation products* | | | | |
|---|---|---|---|---|---|---|---|---|
| | | | | P1* | P2* | P3*** | P4**** | Total |
| Example 1 | 30º C | 0 | 9.94 | - | - | - | - | - |
| | 45º C | 0 | 9.95 | - | - | - | - | - |
| | 60º C | 0 | 9.91 | - | - | - | - | - |
| Example 2 | 30º C | 0 | 9.91 | - | - | - | - | - |
| | 45º C | 0 | 9.65 | - | - | - | - | - |
| | 60º C | 0 | 9.65 | - | - | - | - | - |
| Ref. Ex.1 | 30º C | 0 | 8.28 | - | - | - | - | - |
| | 45º C | 1 | 8.91 | - | - | - | - | - |
| | 60º C | 2 | 8.92 | - | - | - | - | - |
| Perfalgan | 30º C | 1 | 9.78 | 0.02 | - | 0.04 | - | 0.06 |
| | 45º C | 2 | 9.67 | - | - | - | - | - |
| | 60º C | 3 | 9.92 | - | - | - | - | - |

| | | | | | | | | |
|---|---|---|---|---|---|---|---|---|
| * 4-aminophenol not detected (-): <0.002% ** 4-nitrophenol not detected (-): <0.01% *** P3 not detected (-): <0.02% **** P4 not detected (-): <0.005% | | | | | | | | |

Finally, Table VIII shows the results obtained after 6 weeks. It can be observed that the compositions of the invention (Example 1 and Example 2) remain colourless, and only a 0.003% of 4-aminophenol is observed at 60º C for the composition of Example 2. Reference Example 1 shows a higher rate of this product (0.008%) at 60º C, as well as significant colouring at all temperatures. In Perfalgan® samples, the concentrations of 4-aminophenol are still higher (0.12%) and the solution shows important colouring:

**TABLE VIII**

| *Week 6* | *T* | *Colour* | *Paracetamol (mg)* | *Degradation products* | | | | |
|---|---|---|---|---|---|---|---|---|
| | | | | P1* | P2* | P3*** | P4**** | Total |
| Example 1 | 30º C | 0 | 9.88 | - | - | - | - | - |
| | 45º C | 0 | 9.78 | - | - | - | - | - |
| | 60º C | 0 | 9.84 | - | - | - | - | - |
| Example 2 | 30º C | 0 | 9.66 | - | - | - | - | - |
| | 45º C | 0 | 9.55 | - | - | - | - | - |
| | 60º C | 0 | 9.42 | 0.003 | - | - | - | 0.003 |
| Ref. Ex.1 | 30º C | 2 | 9.21 | - | - | - | - | - |
| | 45º C | 2 | 8.68 | - | - | - | - | - |
| | 60º C | 3 | 8.81 | 0.008 | - | - | - | 0.008 |
| Perfalgan | 30º C | 2 | 10.11 | - | - | - | - | - |
| | 45º C | 2 | 9.97 | - | - | - | - | - |
| | 60º C | 4 | 10.04 | 0.12 | - | - | - | 0.12 |

| | | | | | | | | |
|---|---|---|---|---|---|---|---|---|
| * 4-aminophenol not detected (-): <0.002% ** 4-nitrophenol not detected (-): <0.01% *** P3 not detected (-): <0.02% **** P4 not detected (-): <0.005% | | | | | | | | |

According to the results obtained in this test, it can be observed that the compositions of the invention show notable stability, superior to that of the commercial product tested and its equivalent, Reference Product 1, which contain cysteine as antioxidant agent.

### Example 4: Comparative stability testing of the formulations of the invention with respect to formulations without tromethamine

To check the stabilizing effect of tromethamine in the compositions of the invention, the stability of an equivalent composition to those of the invention but without tromethamine was studied, and it was compared with its equivalent containing tromethamine.

Thus, the stability of the composition of Reference Example 2 (with mannitol and citrate buffer, without tromethamine) was tested and the results were compared with those obtained for the composition of Example 2 (with mannitol, citric acid and tromethamine).

A sample of the composition of Reference Example 2 was tested under temperature conditions of 60º C, for 12 days. The samples were analyzed at the beginning and at the end of the test, and the content of paracetamol and of the total of its degradation products was determined by high performance liquid chromatography (HPLC), using the same conditions described in Example 3. A visual analysis of the colouring of the solutions was also performed, in the same way as in Example 3, using the same rating scale from 0 to 4.

Table IX shows the results obtained in this test for Reference Example 2, and they are compared to the results obtained for the composition of Example 2, at the conditions of 60 ºC, at time 0 and at 1, 3 and 6 weeks, according to the data taken from Tables V to VIII of Example 3, respectively.

**TABLE IX**

| *Time* | *Colour* | *Paracetamol (mg)* | *Total degradation products (%)* |
|---|---|---|---|
| *Ref Example 2 (with mannitol, without tromethamine). T=60º C* | | | |
| Beginning | 0 | 9.627 | - |
| 12 days | 2 | 7.145 | 2.08 |

| *Example 2 (with mannitol and tromethamine) T=60º C* | | | |
|---|---|---|---|
| Beginning | 0 | 9.94 | - |
| week 1 | 0 | 9.81 | - |
| week 3 | 0 | 9.65 | - |
| week 6 | 0 | 9.42 | 0.003 |

It can be observed that the composition of Example 2, with tromethamine, remains colourless after 6 weeks, whereas the composition without tromethamine shows colouring already after 12 days. Moreover, the latter shows a 2.08% of degradation products after 12 days, while the composition of the invention shows only a 0.003% of degradation products after 6 weeks.

Therefore, the results of this test allow highlighting the notable stabilizing effect of tromethamine in the aqueous solutions of paracetamol.

## Claims

1. Aqueous composition for injection comprising:
- paracetamol as active ingredient,
- an isotonizing agent,
- an effective amount of a buffering system to obtain a pH comprised between 4.5 and 7.5,
**characterized in that** the buffering system comprises a carboxylic acid and tromethamine.

2. Composition according to claim 1, **characterized in that** the concentration of tromethamine is comprised between 0.1 mg and 3 mg of tromethamine per ml of solution.

3. Composition according to any one of claims 1 or 2, **characterized in that** it has a pH comprised between 5.5 and 6.5.

4. Composition according to any one of claims 1 to 3, **characterized in that** the carboxylic acid is selected from the acids lactic, acetic, sorbic, gluconic, tartaric, malic, succinic, fumaric, citric, and mixtures thereof.

5. Composition according to claim 4, **characterized in that** the carboxylic acid is citric acid.

6. Composition according to claim 5, **characterized in that** it comprises between 0.02 mg and 1.32 mg of citric acid per ml of solution.

7. Composition according to any one of claims 1 to 6, **characterized in that** tromethamine and the carboxylic acid are used in a molar ratio comprised between 1.2:1 and 3:1.

8. Composition according to any one of claims 1 to 7, **characterized in that** the concentration of paracetamol is comprised between 5 mg and 50 mg of paracetamol per ml of solution.

9. Composition according to any one of claims 1 to 8, **characterized in that** the isotonizing agent is selected from sodium chloride, potassium chloride, ethanol, polyethylene glycol, propylene glycol, sorbitol, inositol, mannitol, glycerol, glucose, fructose, dextrose, mannose, sucrose, lactose, maltose, and mixtures thereof.

10. Composition according to any one of claims 1 to 9, **characterized in that** it is packed in vials containing between 0.4 g and 0.6 g of paracetamol per vial.

11. Composition according to claim 1, **characterized in that** it comprises:
- between 5 mg/ml and 50 mg/ml of paracetamol,
- between 0.1 mg/ml and 3 mg/ml of tromethamine,
- between 0.02 mg/ml and 1.32 mg/ml of citric acid, and
- between 1 mg/ml and 15 mg/ml of sodium chloride.

12. Composition according to claim 1, **characterized in that** it comprises:
- between 5 mg/ml and 50 mg/ml of paracetamol,
- between 0.1 mg/ml and 3 mg/ml of tromethamine,
- between 0.02 mg/ml and 1.32 mg/ml of citric acid, and
- between 10 mg/ml and 50 mg/ml of mannitol.

13. Use of tromethamine for the stabilization of injectable aqueous solutions of paracetamol.

14. Process for the preparation of a composition according to any one of claims 1 to 12, **characterized in that** it comprises the following steps:
1) dissolving paracetamol, tromethamine, carboxylic acid and isotonizing agent in water for injection; and
2) adjusting the pH, if necessary, by adding acid or base to a value comprised between 4.5 and 7.5.

15. Composition according to any one of claims 1 to 12 for use in the treatment of pain and fever conditions.

## Patentansprüche

1. Wässrige Zusammensetzung zur Injektion, die Folgendes umfasst:
- Paracetamol als Wirkstoff,
- ein Isotonisierungsmittel,
- eine wirksame Menge eines Puffersystems, um einen pH-Wert zwischen 4,5 und 7,5 zu erhalten,
**dadurch gekennzeichnet, dass** das Puffersystem eine Carbonsäure und Tromethamin umfasst.

2. Zusammensetzung nach Anspruch 1, **dadurch gekennzeichnet, dass** die Tromethamin-Konzentration zwischen 0,1 mg und 3 mg Tromethamin pro ml Lösung umfasst.

3. Zusammensetzung nach einem der Ansprüche 1 oder 2, **dadurch gekennzeichnet, dass** sie einen pH-Wert zwischen 5,5 und 6,5 aufweist.

4. Zusammensetzung nach einem der Ansprüche 1 bis 3, **dadurch gekennzeichnet, dass** die Carbonsäure aus den Säuren Milchsäure, Essigsäure, Sorbinsäure, Gluconsäure, Weinsäure, Apfelsäure, Bernsteinsäure, Fumarsäure, Zitronensäure und Gemischen daraus ausgewählt wird.

5. Zusammensetzung nach Anspruch 4, **dadurch gekennzeichnet, dass** die Carbonsäure Zitronensäure ist.

6. Zusammensetzung nach Anspruch 5, **dadurch gekennzeichnet, dass** sie zwischen 0,02 mg und 1, 32 mg Zitronensäure pro ml Lösung umfasst.

7. Zusammensetzung nach einem der Ansprüche 1 bis 6, **dadurch gekennzeichnet, dass** Tromethamin und die Carbonsäure in einem Molverhältnis zwischen 1,2:1 und 3:1 verwendet werden.

8. Zusammensetzung nach einem der Ansprüche 1 bis 7, **dadurch gekennzeichnet, dass** die Paracetamol-Konzentration zwischen 5 mg und 50 mg Paracetamol pro ml Lösung umfasst.

9. Zusammensetzung nach einem der Ansprüche 1 bis 8, **dadurch gekennzeichnet, dass** das Isotonisierungsmittel aus Natriumchlorid, Kaliumchlorid, Ethanol, Polyethylenglykol, Propylenglykol, Sorbitol, Inosit, Mannitol, Glycerin, Glukose, Fruktose, Dextrose, Mannose, Saccharose, Laktose, Maltose und Gemischen daraus ausgewählt wird.

10. Zusammensetzung nach einem der Ansprüche 1 bis 9, **dadurch gekennzeichnet, dass** sie in Glasfläschchen verpackt wird, die zwischen 0,4 g und 0,6 g Paracetamol pro Glasfläschchen enthalten.

11. Zusammensetzung nach Anspruch 1, **dadurch gekennzeichnet, dass** sie Folgendes umfasst:
- zwischen 5 mg/ml und 50 mg/ml Paracetamol,
- zwischen 0,1 mg/ml und 3 mg/ml Tromethamin,
- zwischen 0,02 mg/ml und 1,32 mg/ml Zitronensäure und
- zwischen 1 mg/ml und 15 mg/ml Natriumchlorid.

12. Zusammensetzung nach Anspruch 1, **dadurch gekennzeichnet, dass** sie Folgendes umfasst:
- zwischen 5 mg/ml und 50 mg/ml Paracetamol,
- zwischen 0,1 mg/ml und 3 mg/ml Tromethamin,
- zwischen 0,02 mg/ml und 1,32 mg/ml Zitronensäure und
- zwischen 10 mg/ml und 50 mg/ml Mannitol.

13. Verwendung von Tromethamin zur Stabilisierung der injizierbaren wässrigen Paracetamollösungen.

14. Verfahren zur Herstellung einer Zusammensetzung nach einem der Ansprüche 1 bis 12, **dadurch gekennzeichnet, dass** es die folgenden Schritte umfasst:
1) Auflösung von Paracetamol, Tromethamin, Carbonsäure und des Isotonisierungsmittels in Wasser zur Injektion; und
2) Anpassung des pH-Werts bei Bedarf durch Zusatz einer Säure oder Base auf einen Wert zwischen 4,5 und 7,5.

15. Zusammensetzung nach einem der Ansprüche 1 bis 12 zur Verwendung bei der Behandlung von Schmerz- und Fieberzuständen.

## Revendications

1. Composition aqueuse pour injection comprenant :
- du paracétamol en tant que principe actif,
- un agent d'isotonicité,
- une quantité efficace d'un système tampon pour obtenir un pH compris entre 4,5 et 7,5,
**caractérisée en ce que** le système tampon comprend un acide carboxylique et de la trométhamine.

2. Composition selon la revendication 1, **caractérisée en ce que** la concentration de la trométhamine est comprise entre 0,1 mg et 3 mg de trométhamine par ml de solution.

3. Composition selon l'une quelconque des revendications 1 ou 2, **caractérisée en ce qu'**elle a un pH compris entre 5,5 et 6,5.

4. Composition selon l'une quelconque des revendications 1 à 3, **caractérisée en ce que** l'acide carboxylique est choisi parmi les acides lactique, acétique, sorbique, gluconique, tartrique, malique, succinique, fumarique, citrique, et leurs mélanges.

5. Composition selon la revendication 4, **caractérisée en ce que** l'acide carboxylique est l'acide citrique.

6. Composition selon la revendication 5, **caractérisée en ce qu'**elle comprend entre 0,02 mg et 1,32 mg d'acide citrique par ml de solution.

7. Composition selon l'une quelconque des revendications 1 à 6, **caractérisée en ce que** la trométhamine et l'acide carboxylique sont utilisés dans un rapport molaire compris entre 1,2/1 et 3/1.

8. Composition selon l'une quelconque des revendications 1 à 7, **caractérisée en ce que** la concentration du paracétamol est comprise entre 5 mg et 50 mg de paracétamol par ml de solution.

9. Composition selon l'une quelconque des revendications 1 à 8, **caractérisée en ce que** l'agent d'isotonicité est choisi parmi du chlorure de sodium, du chlorure de potassium, de l'éthanol, du polyéthylène glycol, du propylène glycol, du sorbitol, de l'inositol, du mannitol, du glycérol, du glucose, du fructose, du dextrose, du mannose, du saccharose, du lactose, du maltose, et leurs mélanges.

10. Composition selon l'une quelconque des revendications 1 à 9, **caractérisée en ce qu'**elle est conditionnée dans des flacons contenant entre 0,4 g et 0,6 g de paracétamol par flacon.

11. Composition selon la revendication 1, **caractérisée en ce qu'**elle comprend :
- entre 5 mg/ml et 50 mg/ml de paracétamol,
- entre 0,1 mg/ml et 3 mg/ml de trométhamine,
- entre 0,02 mg/ml et 1,32 mg/ml d'acide citrique, et
- entre 1 mg/ml et 15 mg/ml de chlorure de sodium.

12. Composition selon la revendication 1, **caractérisée en ce qu'**elle comprend :
- entre 5 mg/ml et 50 mg/ml de paracétamol,
- entre 0,1 mg/ml et 3 mg/ml de trométhamine,
- entre 0,02 mg/ml et 1,32 mg/ml d'acide citrique, et
- entre 10 mg/ml et 50 mg/ml de mannitol.

13. Utilisation de trométhamine pour la stabilisation de solutions aqueuses injectables de paracétamol.

14. Procédé de préparation d'une composition selon l'une quelconque des revendications 1 à 12, **caractérisé en ce qu'**il comprend les étapes suivantes :
1) la dissolution du paracétamol, de la trométhamine, de l'acide carboxylique et de l'agent d'isotonicité dans de l'eau pour injection ; et
2) l'ajustement du pH, si nécessaire, par l'addition d'un acide ou d'une base jusqu'à une valeur comprise entre 4,5 et 7,5.

15. Composition selon l'une quelconque des revendications 1 à 12 pour une utilisation dans le traitement d'affections douloureuses et fébriles.
